# EUROPEAN PATENT APPLICATION

(11) **EP 1 595 545 A1**
(43) Date of publication of application: **16.11.2005**
(21) Application number: 04711734.6
(22) Date of filing: 17.02.2004
(51) Int. Cl.: A61K 31/616, A61P 9/00, A61P 43/00

(54) **BLOOD-VESSEL-GROWTH PROMOTER**

(30) Priority: 21.02.2003 JP 2003044392
(71) Applicant: TEIKOKU SEIYAKU CO., LTD., Higashikagawa-shi, Kagawa 769-2695 (JP)
(72) Inventor: INAMOTO, Yukiko, 7611703 (JP); KAWADA, Mitsuhiro, 7692702 (JP); TSUKAMOTO, Ikuko, 7610701 (JP); KUBOTA, Yasuo, 7600074 (JP)
(74) Representative: Albrecht, Thomas
(86) International application number: PCT/JP2004/001700
(87) International publication number: WO 2004/073717

(57) **Abstract**

A neo-vascularization promoter containing acetylsalicylic acid or its pharmaceutically acceptable salt as an active ingredient.

## Description

### TECHNICAL FIELD

The present invention relates to a preparation containing acetylsalicylic acid as an active ingredient having excellent neo-vascularization promoting activity, and a method for treating a patient suffering from a disease caused by lack of neo-vascularization which comprises administering said preparation.

### BACKGROUND ART

Neo-angiogenesis is a phenomenon that a small blood vessel is newly formed from an already existed blood vessel. The prognosis of the circular diseases such as myocardic infarction is affected by many factors, and the degree of the development of collateral vessels is considered to be one of the most important determining-factors of the convalescence. When collateral vessels are sufficiently developed, even if arctation or infarction occurs, ischemia or necrosis of the tissue is inhibited, and size of the infarction decreases or the convalescence is improved.

From of old as a mechanism of collateral vessel-formation, change of the inner pressure of the blood vessel and hemokinesis (blood flow) has been made a big point, but it was reported that in vascular endothelial cells and vascular smooth muscular cells, cytodieresis figure associated with DNA synthesis was recognized in case of formation of collateral vessel. Therefore, it becomes to be recognized that the process of collateral vessel-formation is not only expansion due to physical factors of the already existed inosculated vessel, but also at least a part of the process is neo-vascularization which proliferation of cells composing blood vessel wall participates.

On the other hand, neo-vascularization is widely confirmed in the physiological phenomenon such as wound healing. Its mechanism is considered to be due to migration or proliferation of vascular endothelial cells from venula or capillary which is already existed, or due to tube formation. Furthermore, interactions via cytokine or growth factor among other cells (fibroblast, smooth muscular cells) surrounding vascular endothelial cells is also considered to be an important factor in regard to wound healing.

Nowadays, on substances having neo-vascularization promoting activity such as fibroblast growth factor (FGF), hepatocyto growth factor (HGF), etc. and neo-vascularization promoting factor such as vascular endothelial cell growth factor (VEGF), etc., had been evaluated the possibility as an agent for treating an angiogenic disease, and the results were reported (See Internal medicine, Vol. 85, No. 5. page 893-897 (May, 2000), Internal medicine, Vol. 85, No. 5. page 886-892 (May, 2000), etc.). However, these substances have not been in practice and the development of the excellent substances is still desired.

### DISCLOSURE OF INVENTION

The object of the present invention is to provide an agent for treating a disease caused by lack of neo-vascularization having excellent neo-vascularization promoting activity with less side effect.

The present invention relates to a neo-vascularization promoter (hereinafter abbreviate as a neo-vascularization promoter of the present invention or a drug of the present invention.) containing acetylsalicylic acid or its pharmaceutically acceptable salt as an active ingredient.

The present invention relates to a neo-vascularization promoter containing an amount of 0.01 to 80% by weight of acetylsalicylic acid or its pharmaceutically acceptable salt.

The present invention relates to a method for treating a patient suffering from a disease caused by lack of neo-vascularization disease which comprises administering said preparation to the patient in an effective amount of it.

The present inventors have extensively studied to dissolve the above problems, and have found that acetylsalicylic acid or its pharmaceutically acceptable salt unexpectedly showed strong neo-vascularization promoting activity. Thus the present invention was completed.

### BEST MODE FOR CARRYING OUT THE INVENTION

The present inventors confirmed that proliferation activity of human umbilical vein endothelial cells (HUVEC) was shown by adding acetylsalicylic acid to HUVEC in vitro test.

Furthermore, in vitro test using a mixture of human vascular endothelial cell and fibroblast by adding acetylsalicylic acid thereto, promoting activity of tube formation was shown and further in vivo test on neo-vascularization by using rats, neo-vascularization promoting activity was shown by administration of acetylsalicylic acid.

This effect depends on the concentration of acetylsalicylic acid in a preparation, but the efficacy is not changed even beyond a certain amount.

The neo-vascularization promoter of the present invention is usually administered systematically, locally, orally or parenterally.

The amount and administration route of the neo-vascularization promoter of the present invention are not specially limited, but the most suitable amount is determined in accordance with the administration route, ages, sex, severity, and frequency, body weight, etc.

In case of the systemic application such as injection, oral application or insertion of suppository, the amount is 0.01 to 4.5g/adult/day once or more times a day.

As the neo-vascularization promoter of the present invention, acetylsalicylic acid or its pharmaceutically acceptable salt itself may be used, but a preparation prepared by blending it with fillers used in usual preparation, or other additives may be used.

The blood concentration of acetylsalicylic acid contained in the preparation of the present invention is 1ng/mL to 550µg/mL (0.0005 to 3000µM), preferably 10ng/mL to 55µg/mL, more preferably 100ng/mL to 25µg/mL. When the concentration of acetylsalicylic acid is less than 1ng/mL, the effect is not enough, and when beyond 550µg/mL, there is a high possibility to occur side effect.

The tissue concentration of acetylsalicylic acid contained in the preparation of the present invention is 0.01ng/g to 1000µg/g, preferably 0.1ng/g to 500µg/g, more preferably 1ng/g to 100µg/g. When the tissue concentration of acetylsalicylic acid is less than 0.01ng/g, the effect is not enough, and when beyond 1000µg/g, there is a high possibility to occur side effect.

The acetylsalicylic acid as an active ingredient of the present invention is a pharmaceutically acceptable amino acid salt such as lysine salt or an inorganic salt such as sodium salt as well as acetylsalicylic acid.

The preparation of the present invention is any preparation such as a solid preparation, a liquid composition or other composition suitable for oral administration, parenteral application such as injection, suppository, and if necessary a suitable preparation is selected.

As an oral preparation, tablets, pills, capsules, powders, granules, solutions, etc. can be illustrated.

On the other hand, as an external preparation, as long as the preparation can be applied directly to the local surface of the skin, it is not specifically limited, such as preparation such as ointments, creams, gels, patches, solutions (suspensions, emulsions, lotions, etc.), cataplasms, tapes, external powders, aerosols, etc.

The amount of acetylsalicylic acid or its pharmaceutically acceptable salt contained in the preparation depends on the form of the preparation, but the amount is 0.005 to 80% by weight per total weight which shows enough effect, preferably 0.01 to 70% by weight, more preferably 0.01 to 50% by weight. When the amount of acetylsalicylic acid contained therein is less than 0.005% by weight, it is not preferable because the activity of acetylsalicylic acid is not shown enough, and when beyond 80% by weight, it is difficult to prepare the preparation.

The diseases directed to the present invention is neo-vascularization together with repairing of the tissue, for example, temperature disturbance such as fire injury, heat injury, heat injured ulcus, frostbite, etc.; external injury such fracture, abrasion, incise wound, bite, acne, bite, etc.; blood vessel or lymphotube injury such as Bürger disease, lymphedemas, crus ulcer, etc.; postoperative wound, such as donor site, sutural etc.; dermal wound such as decubitus, compressive ulcer, diabetic ulcer·gangrene, stoma, radiation injury, chemical injury, etc.; dermal injury such as blister, erosion etc.; ischemic disease (circular disease), such as myocardiac infarction, etc.

### Example

The present invention is explained by test-examples and preparation-examples by using acetylsalicylic acid, but the present invention should not be limited by these examples.

### Test

The neo-vascularization promoting activity of the present invention was evaluated in vitro test.

### Test 1

### Proliferation test on human umbilical vein endothelial cells (HUVEC)

In a plate having 96 wells a certain amount (2000-4000 cells) of HUVEC were seeded, and the cells were cultured in the CS-C broth containing 5% fetal bovine serum (Cell Systems Co.) over night to adhere thereto. After the culture broth was filtered by suction, to a minimum essential medium (MEM) broth containing 1% FBS, acetylsalicylic acid was added at a concentration (0, 0.055, 0.55, 5.5, 55, 555µM, respectively) and each mixture was cultivated. After 0, 24 and 48 hours the number of alive cells was counted. As a positive control vascular endothelial growth factor (VEGF; Vascular Endothelial Growth Factor-A) (20ng/mL) was used. In this measurement, Cell Counting Kit-8 (Dojin Chemical Co.) was used.

The result was shown in Table 1.

**Table 1**

| Change of the number of HUVEC | | | | |
|---|---|---|---|---|
| Group (drug) | Conc. of drug (µM) | Relative absorvancy | | |
| | | After 0 hour | After 24 hours | After 48 hours |
| Control | - | 1.000 | 1.236 | 1.200 |
| VEGF | 20ng/mL | 1.003 | 2.041 | 1.889 |
| Acetylsalicylic acid | 0.055 | 1.102 | 1.252 | 1.303 |
| | 0.55 | 1.144 | 2.001 | 1.709 |
| | 5.5 | 1.084 | 1.811 | 1.575 |
| | 55 | 1.107 | 1.754 | 1.475 |
| | 555 | 1.017 | 1.044 | 0.699 |

From the result shown in Table 1, when acetylsalicylic acid was added in more than 0.055µM, proliferation promoting activity of HUVEC was confirmed and this effect was nearly equal to the effect of vascular endothelial growth factor (VEGF). When acetylsalicylic acid was added in the amount of more than 555µM, there showed the tendency that its proliferation promoting effect was not recognized or was even depressed.

### Test 2

### Measurement of neo-vascularization ability

The tube formation by a mixture-system of human vascular endothelial cells (HUVEC) and fibroblast was investigated by CD31 staining kit (Kurabo Ind.) to be visualized, and be made score by an analytical soft (NIH Image). A broth containing acetylsalicylic (0.5, 5.5, 55µM; changed every three days) was added to neo-vascularization kit (Angiogenesis Kit KZ-1000; Kurabo Ind.) prepared by mixing human vascular endothelial cells and fibroblast, and the resulting broth was cultivated for 11 days. Then immuno histochemical study was performed by using CD31 staining kit, and the color photo was taken. The picture was analyzed using NIH Image and the amount of the formed tubes was scored as an area to evaluate. As a positive control, vascula endothelial growth factor (VEGF; 10ng/mL) and as a negative control sramine (100µM) were used, respectively.

The result was shown in Table 2.

**Table 2**

| Neo-vascularization ability | | |
|---|---|---|
| Group | Conc. of drug (µM) | Relative area |
| | | After 11 days |
| Control | - | 1.00 |
| VEGF | 10ng/mL | 1.71 |
| Sramine | 100 | 0.04 |
| Acetylsalicylic acid | 5.5 | 1.17 |
| | 55 | 1.08 |

From the result shown in Table 2, the group containing acetylsalicylic acid showed tube-formation promoting activity.

### Test example 3

### Neo-vascularization test using rats

Wister female rats (7 weeks old; n=6) were anesthetized with ether, and a urethane sponge (diameter: 8mm) previously permeating a drug was inserted in subcutaneous tissue of right shoulder. As a test drug, acetylsalicylic acid (2mg) and as a positive control retinoid (0.5mg) were administered, respectively. Four days later after insertion of the sponge, 5% gelatin solution containing 5% carmine dye kept at 37°C was administered under ether anesthesia in tail vein of the rat. After injection, the rats in suspended animation were left at 4°C for 2 hours, and the gelatin solution containing carmine dye distributed in blood vessel was solidified. After butcher of the rats, the sponge with granulation tissue was extracted and the dye therein was extracted. Absorbancy at 530nm was measured to calculate the amount of the carmine dye.

The result was shown in Table 3.

**Table 3**

| Amount of carmine dye | |
|---|---|
| Test group | Carmine dye (µg/mL) |
| Acetylsalicylic acid | 16.0 ± 4.2 |
| Retinoid | 27.1 ± 6.8 |
| Control | 9.2 ± 5.7 |

From the result shown in Table 3, as the amount of carmine dye was much more than control, it was confirmed that neo-vascularization was promoted.

As mentioned above, in the test in vitro using HUVEC, etc., the effect of acetylsalicylic acid on cell proliferation and tube-formation was tested, and as a result proliferation and tube-formed activity of HUVEC was confirmed. Therefore, it was found that the preparation of the present invention was very useful as a neo-vascularization promoter.

### Preparation example 1

### Tablets

Acetylsalicylic acid (100 parts by weight), lactose (200 parts by weight), starch (50 parts by weight), crystalline cellulose (147 parts by weight) and magnesium stearate (3 parts by weight) were blended and the mixture was tableted to prepare tablets weighing 100mg per tablet and containing acetylsalicylic acid 20mg per tablet.

### Preparation example 2

### Capsules

Acetylsalicylic acid (200mg), microcrystalline cellulose (400mg), silicon dioxide (10mg) and magnesium stearate (5mg) were added in hard capsules to prepare capsules.

### Preparation example 3

### Suppositories

Sucrose fatty acid ester (50mg) was dispersed in a part of hard fat and the mixture was melted by heating at more than 90°C. After acetylsalicylic acid (750mg) and the rest of hard fat were dispersed at 60°C, the mixture was well blended with the previously prepared solution. The mixture was filled in a vessel for suppositories and cooled to solid to give suppositories of total weight 2500mg.

### Preparation example 4

### Ointments

Hydrocarbon gel (93 parts by weight) and isopropyl adipate (5 parts by weight) were mixed under heating, and thereto was added acetylsalicylic acid (2 parts by weight). The mixture was well kneaded under agitating to prepare ointments.

### Preparation example 5

### Tapes

Stylene-isoprene-stylene block copolymer (30 parts by weight), hydrogenated rosin glycerin ester (25 parts by weight), polybutene (9 parts by weight) and dibutyl hydroxytoluene (1 part by weight) were put in a kneader under warming, and the mixture was stirred under warming to melt it. Separately, acetylsalicylic acid (10 parts by weight), isopropyl myristate (10 parts by weight) and hydrogenated rosin glycerin ester (15 parts by weight) were mixed and stirred, and the mixture was added to the mixture previously prepared. The mixture was completely kneaded and the ointment base was spread on cloth to cut in desired size to prepare tapes.

### Preparation example 6

### Powders

Potato starch (76 parts by weight), zinc oxide (4 parts by weight) and acetylsalicylic acid (20 parts by weight) were mixed until it became completely homogenously to prepare powders.

### INDUSTRIAL APPLICABILITY

It is expected that the neo-vascularization promoter of the present invention containing acetylsalicylic acid or its pharmaceutically acceptable salt as an active ingredient shows excellent therapeutic effect against, angiogenic diseases required for neo-vascularization (temperature disturbance such as fire injury, heat injury, heat injured ulcus, frostbite, etc.; external injury such fracture, abrasion, incise wound, bite, acne, bite, etc.; blood vessel or lymphotube injury such as Bürger disease, lymphedemas, crus ulcer, etc.; postoperative wound, such as donor site, sutural, etc.; dermal wound such as decubitus, compressive ulcer, diabetic ulcer·gangrene, stoma, radiation injury, chemical injury, etc.; dermal injury such as blister, erosion, etc.; ischemic disease (circular disease) such as myocardiac infarction, etc.).

## Claims

1. A neo-vascularization promoter containing acetylsalicylic acid or its pharmaceutically acceptable salt as an active ingredient.

2. The neo-vascularization promoter according to claim 1 wherein acetylsalicylic acid or its pharmaceutically acceptable salt is contained in an amount of 0.01 to 80% by weight.

3. A method for promoting neo-vascularization administering to a patient suffering from disease caused by lack of neo-vascularization an effect amount of acetylsalicylic acid or its pharmaceutically acceptable salt.
